Europäisches Patentamt

European Patent Office

Office européen des brevets

(11) Publication number: 0 210 956
A2

(12) EUROPEAN PATENT APPLICATION

(21) Application number: 86830138.3

(22) Date of filing: 26.05.86

(51) Int. Cl.⁴: **A 61 M 11/02**

(30) Priority: 29.07.85 IT 2264585 U

(43) Date of publication of application:
04.02.87 Bulletin 87/6

(84) Designated Contracting States:
AT BE CH DE FR GB LI LU NL SE

(71) Applicant: MICROMAX S.p.A.
Via Monterosa 7
I-22079 Villaguardia (Como)(IT)

(72) Inventor: Giannelli, Giuseppe
Parco Vignazze
I-22077 Olgiate Comasco Como(IT)

(72) Inventor: Bologna, Italo
Via Carovelli, 42
I-22075 Lurate Caccivio Como(IT)

(74) Representative: Perani, Aurelio et al,
c/o JACOBACCI-CASETTA & PERANI S.p.A 7, Via Visconti
di Modrone
I-20122 Milano(IT)

(54) An electric nebulizer household appliance for cosmetic treatment.

(57) An electric nebulizer household appliance comprises a boiler body (1) having a vaporizing chamber (29) in fluid communication with a steam delivery spout (4). The steam delivery spout (4) is connected to the vaporizing chamber (29) by a flexible hose (60), is provided with a handle (62) and is supported removably on the boiler body (1).

Fig-1

EP 0 210 956 A2

0210956

DESCRIPTION

This invention relates to an electric nebulizer household appliance of a type which comprises a boiler body having a vaporizing chamber defined therein, and a steam delivery spout mounted in fluid communication with said vaporizing chamber. Apparatus of this general type are widely used for cosmetic treatment of the face and other parts of the human body.

Conventionally, these are constructed to include a boiler body with an immersed electric resistance heater for steam generating, and a steam delivery spout attached to said boiler body close to the top thereof.

Such prior appliances are somewhat unpractical to use on account of the orientation of the steam jet from the delivery spout being set fixedly and the user compelled to so adjust his/her position as to let a part of the body to be treated to be swept by the steam.

Some professional type appliances, intended for use at beauty parlors, have their delivery spout mounted to the tip of an adjustable arm, so as to obviate such drawbacks at least in part.

On the other hand, such professional type appliances are bulky and expensive, and accordingly, unsuitable for domestic use.

It is the object of this invention to provide an electric nebulizer household appliance which is structurally and operatively configured to overcome the aforementioned prior disadvantages.

This object is achieved by an electric household appliance as indicated, which is characterized in that the steam delivery spout is connected to the vaporizing chamber by means of a flexible hose, provided with a handle, and associated with said boiler body in a removable fashion.

The invention features and advantages will be better understood by making reference to the following detailed description of a preferred embodiment thereof, shown by way of example and not of limitation in the accompanying drawings, where:

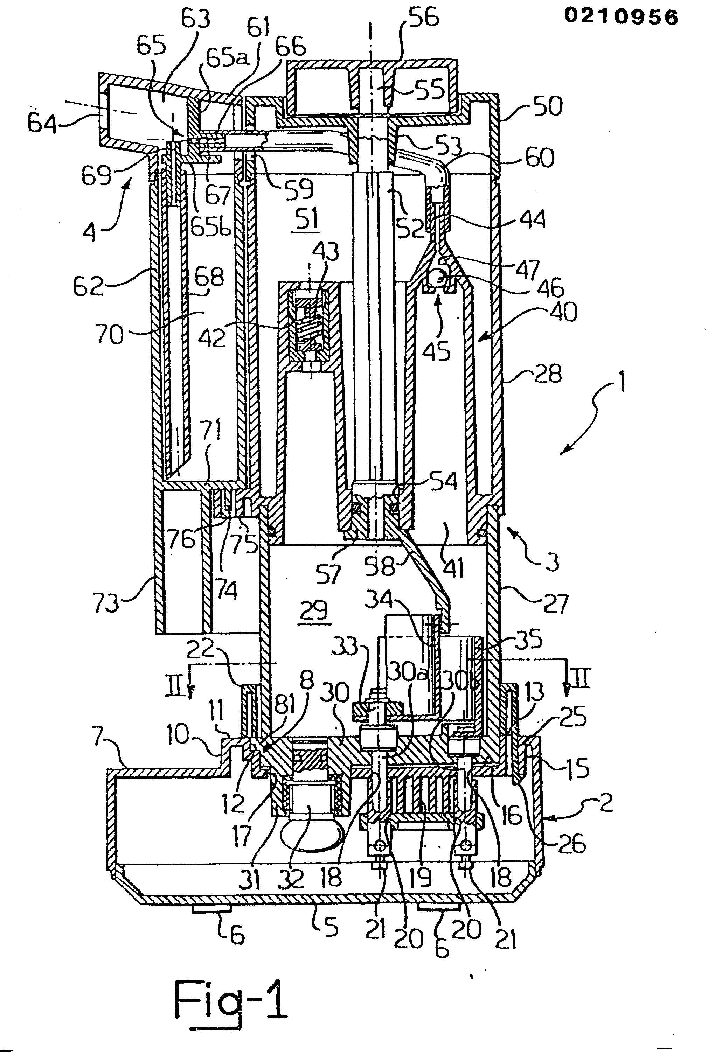

Figure 1 is a longitudinal section view of an electric household appliance according to this invention;

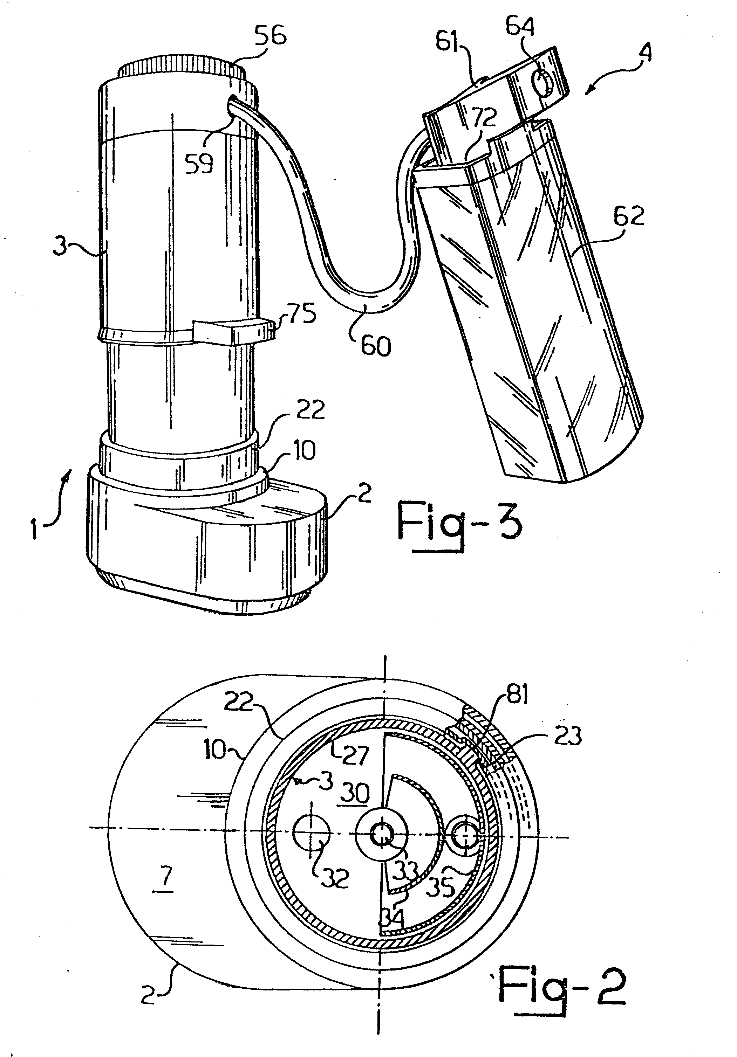

Figure 2 is a sectional view of the appliance of Figure 1, taken along the line II-II; and

Figure 3 is a perspective view of the appliance of Figure 1, as with the delivery spout removed from the boiler body.

In the accompanying drawings, the reference numeral 1 designates generally an electric household appliance according to the invention. The appliance 1 comprises a base 2, boiler body 3, and delivery spout 4.

The base 2 is of hollow interior construction and has a flattened cylindrical shape with an oval cross-section. It has a substantially flat bottom face 5 provided with small feet 6, and a top face 7 axially opposing the bottom face, wherein a seat 8 is formed for accommodating the bottom end of the boiler body 3 therein.

The seat 8 is encircled by an annular step 10 having a top

surface 11 and a series of windows 13 extending along an arc of a circle and being spaced apart from one another.

A lip 15 extends from the surface 11 toward the bottom face 5 at the outboard edge of each window 13.

Inwardly of the step 10, the seat 8 is closed by a substantially flat bottom 16 wherein an opening 17 and a pair of holes 18 are formed.

At the hole pair 18, the bottom 16 is made fast with a mount 19 carrying two female studs 20 and related clamps 21.

A ring nut 22 is mounted rotatably on the surface 11 of the step 10. The ring nut 22 is formed with a depression 23 extending axially in the portion of its skirt facing the seat 8 interior, and with a series of lugs 25 extending through the windows 13. The lugs 25 have their free ends formed into a dog 26 for snap--action engagement over the lip 15 to hold the ring nut 22 onto the base 2.

The boiler body 3 comprises first and second cylindrical portions 27 and 28, respectively, having a circular cross-section.

The portion 27 is of bowl-like shape and bounds a vaporizing chamber 29. It has a bottom 30 wherein a spigot 31 is formed for filling the vaporizing chamber 29 with water.

The spigot 31 is normally closed by a screw-in stopper 32.

The bottom 30 is also through-penetrated by first and second male pins indicated at 30a and 30b, respectively, intended for removable coupling with the female studs 20 with the portion 27 received into the seat 8.

The first male pin 30a is located at the center of the bottom 30 and has a tang 33 extended into the vaporizing chamber 29. The tang 33 forms a rotary carrier pin for a first electrode 34.

A second electrode 35 is attached to the pin 30b. The electrodes 34 and 35 have their active surfaces configured as semicircular bands held apart from each other coaxially with the tang 33.

A detent 81 protrudes radially from the bottom 30 outward of the portion 27. The detent 81 is received into the recess 12 when the bottom 30 is engaged in the seat 8.

On the free end of the portion 27 there is secured in sealed engagement relationship the portion 28 of the boiler body 3.

In side the portion 28, there is formed a cylindrical annular elevation 40 which is coaxial with the boiler body 3 and bounds, on the side next to the portion 27, an annular chamber opening into the vaporizing chamber 29.

Formed in the elevation 40 is a seat 42 for a safety valve 43, known per se.

A conduit 44 is provided at the top of the elevation 40 for delivering the steam generated within the chamber 29.

The conduit 44 incorporates a one-way valve 45 with a spherical shutter 46 freely movable and held in a seat 47 formed integrally at the top of the elevation 40.

The portion 28 is closed at the top by a cover 50 which delimits a compartment 51 therein in cooperation with the elevation 40.

A small shaft 52 is supported rotatably in a first bush 53, formed integrally with the cover 50, and in a second bush 54 formed integrally and centrally in the elevation 40. The shaft 52 is coaxial with the boiler body 3 and has a top end 55, projecting outward of said body 3, onto which a knob 56 is keyed, and a bottom end 57 engaged sealingly in the bush 54.

The shaft 52 is made rotatively rigid with the electrode 34 by an arm 58 which extends from the bottom end 57 into the vaporizing chamber 29.

Formed in the cover 50 is a radial hole 59 wherethrough a flexible hose 60 is passed loosely.

One end of the hose 60 fits tightly over the conduit 44, whilst its opposed end is connected to the steam delivery spout 4 in a manner to be explained hereinafter.

The delivery spout 4 has a body 61 which fits over a handle 62 cap-like fashion.

The body 61 has an internal cavity 63 which opens to the outside through a hole 64 and in which a bracket 65 is secured having two wings 65a and 65b at right angles.

Attached to the wing 65a is a nozzle 66 wherethrough a steam delivery hole 67 extends.

The corresponding end of the hose 60 fits over the nozzle 66.

Attached to the wing 65b is a nosepiece 69 which is drilled axially through and directed perpendicularly to the hole of the nozzle 66. The nosepiece 69 is connected to a dip tube 68.

The handle 62 has an internal cavity 70 enclosing the dip tube 68; the cavity 70 forms a flacon whereinto cosmetic substances can be introduced for nebulization in intimate admixture to the steam generated in the boiler body 3.

The cavity 70 is closed at the bottom by a wall 71, and is provided at the top with two openings 72 flanking the body 61 for introduction of the aforesaid cosmetic substances.

A skirt 73 extends from the wall 71 as a continuation of the side walls of the handle 62. From the wall 71 there also extends a short lug 74.

A bracket 75 extends radially from the boiler body 3; the bracket 75 is formed with a slot 76 adapted to receive the lug 74 removably therein.

The appliance 1 operates as follows: water to be vaporized is introduced into the vaporizing chamber 29 through the spigot 31 after removing the stopper 32. To accomplish this, the boiler body 3 would be removed from the base 2 and turned upside down.

With the boiler body 3 upside down to fill it with water, the one-way valve 45 is closed by the spherical shutter 46 preventing water from flowing into the conduit 44.

The boiler body 3 is then re-installed on the base 2, after turning the ring nut 22 until the depression 23 mates with the recess 12.

The bottom 30 of the boiler body 3 is then positioned into the seat 8 with the detent 81 received in the aforesaid recess 12. The ring nut 22 is again turned to lock the body 3 on the base 2.

The male pins 30a and 30b are coupled with the female studs 20 to supply electric power to the respective electrodes 34 and 35.

Steam is generated within the vaporizing chamber 29 by the current flowing through the water contained in it.

The amount of the steam generated is controlled by means of the knob 56, which enables the electrode 34 to be rotated relatively to the electrode 35 to vary the areas of the confronting surfaces thereof.

The steam generated flows up into the annular chamber 41, whence it is passed into the flexible hose 60 through the conduit 44.

The length of the flexible hose 60 is selected to enable removal of the steam delivery spout 4 and its handle 62 from the boiler body 3, and to conveniently direct the steam jet delivered to the body parts to be treated.

In flowing out through the nozzle 66, the steam will suck up the fluid cosmetic substances contained in the cavity 70 of the handle 62 through the dip tube 68.

After use of the appliance 1, the handle 62 and its spout 4 are stowed on the boiler body 3 with the lug 74 engaged in the slot 76 of the bracket 75.

The hose 60 is coiled up into the compartment 51.

The appliance 1 would be formed preferably by molding from plastics throughout, except for the electrodes 34 and 35, and the male pins and female studs and valve means.

The appliance of this invention achieves the object set forth and affords a number of advantages over conventional appliances.

Among these advantages, noteworthy are the handling convenience of the electric household appliance described above, and the possibility of orienting the steam delivery spout 4 over the body parts awaiting treatment without the boiler body 3 requiring manipulation or shifting.

## CLAIMS

1. An electric nebulizer household appliance comprising a (1) (29) boiler body wherein a vaporizing chamber is defined and a steam (4) (29) delivery spout in fluid communication with said vaporizing chamber, (4) characterized in that the steam delivery spout is connected to (29) (60) the vaporizing chamber by a flexible hose, is provided with a (62) (1) handle, and is associated removably with said boiler body.

2. An appliance according to Claim 1, characterized in that (4) (62) said delivery spout and its related handle are supported removably (1) on said boiler.

3. An appliance according to Claim 1, characterized in that (62) (68) said handle is hollow inside and forms a flacon wherein a dip tube (69) (69) is provided with a nosepiece at one end; said nosepiece being (66) secured in the vicinity of a steam delivery nozzle located in (4) (29) said spout and connected to the vaporizing chamber through said (60) flexible hose.

Fig-1

Fig-3

Fig-2